# EUROPEAN PATENT SPECIFICATION

(11) **EP 0 444 931 B1**
(45) Date of publication and mention of the grant of the patent: **10.08.1994**
(21) Application number: 91301646.5
(22) Date of filing: 28.02.1991
(51) Int. Cl.: A61K 31/28, A61K 31/80

(54) **Use of 3-oxygermylpropionic acid to treat and prevent diabetes due to autoimmune diseases**
Verwendung der 3-Oxygermylpropionsäure zur Behandlung und Prävention von Diabetes verursacht durch Autoimmunkrankheiten
Utilisation de l'acide 3-oxygermylpropionique pour le traitement et la prevention du diabète provoqué par des maladies autoimmunes

(30) Priority: 28.02.1990 JP 48644/90
(43) Date of publication of application: 04.09.1991
(73) Proprietor: SANWA KAGAKU KENKYUSHO CO., LTD., Higashi-ku Nagoya-shi Aichi-ken (JP)
(72) Inventor: Sawai, Kiichi, c/o Sanwa Kagaku Kenkyusho Co., Ltd, Higashi-ku, Nagoya-shi, Aichi-ken (JP); Kurono, Masayasu, c/o Sanwa Kagaku, Higashi-ku, Nagoya-shi, Aichi-ken (JP); Mitani, Takahiko, c/o Sanwa Kagaku, Higashi-ku, Nagoya-shi, Aichi-ken (JP)
(74) Representative: Diamond, Bryan Clive

(56) References cited:
- EP-A- 0 016 444
- EP-A- 0 186 505
- JP-A- 6 041 472
- PATENT ABSTRACTS OF JAPAN, vol. 11, no. 393 (C-466)[2345], 25th December 1987;& JP-A-62 161 724 (SANWA KAGAKU KENKYUSHO K.K.) 17-07-1987
- CHEMICAL ABSTRACTS, vol. 103, no. 9, 2nd September 1985, page 549, abstract no.70130m, Columbus, Ohio, US; & JP-A-60 41 472 (REFORM SYSTEM K.K.) 05-03-1985

## Description

The present invention relates to the use for treating and preventing diabetes mellitus due to autoimmune diseases, of 3-oxygermylpropionic acid (3-OGP) of the formula:

[(O_{1/2})₃GeCH₂CO₂H]ₙ (I)

wherein n represents an integer of 1 or more.

The pharmacological activity of this compound, because it has sophisticated polymerizability and a variety of applications, has recently attracted attention. It has been suggested that it is usable for treating hepatopathy. However, a serious problem with 3-OGP is that it is so unstable in the presence of water that its pharmacological activity decreases drastically (see JP-A-57-53800 and US-A-4309412).

To overcome this defect, we have already found that a variety of substances can be effectively used as stabilizers for 3-OGP (see JP-A-61-65819) and that saccharides can enhance its activity (see JP-A-60-190714). We have also discovered that 3-OGP has immunity-accommodative actions (see JP-A-61-151123).

Patients with debilitated blood sugar-accommodative functions, especially those with diabetes, are likely to have toxic symptoms due to their antidotal hypometabolism or immunodeficiency and so suffer disorders such as allergy, anaphylaxis, cataract, cutaneous ulcers and acute insufficiency, or in the worst case to die. Such disorders are considered generally to be due to autoimmune diseases.

We have now found that 3-OGP, which is of great safety, is efficacious against diabetes mellitus due to autoimmune disease.

More specifically, the present invention provides the use of 3-OGP for the manufacture of a medicament for treating or preventing diabetes mellitus due to autoimmune diseases; the medicament used contains 3-OGP as an active ingredient, in combination with a stabilizing carrier.

Preferably, the stabilizing carrier is hydroxypropylcellulose, which can be used in an amount of 2 parts per 1 part by weight of the active ingredient (3-OGP).

3-OGP has physicochemical properties expressed in terms of a specific gravity or density of 2.23, a solubility in water of 1.57 at 20°C and a melting or decomposition point of about 230°C.

3-OGP can be a polymer, of polymerizability so sophisticated that it can become very unstable in the presence of moisture, thus resulting in a sharp drop in its pharmacological activity. For that reason, we add thereto the stabilizing carrier.

The compositions containing 3-OGP are usually provided in the form of orally administered tablets or capsules which are absorbed through the digestive tract.

Although the dose varies dependent upon the type of compound, the type of composition, the age of patient and other factors, the composition used in this invention may generally be administered to humans at a dose between 5 mg/kg and 1500 mg/kg. A preferable daily dose to adult patients (weight 50 kg) is about 150 mg/kg.

### EXAMPLES

The present invention will now be illustrated by the following examples.

### Preparation Example 1

Hydroxypropylcellulose as carrier and 3-OGP represented by Formula I were mixed together at a weight ratio 2:1. The mixture was kneaded, with ethanol serving as a wetting agent, and then dried at a temperature lower than 50°C to obtain a powdery or granular primary composition.

### Preparation Example 2

The primary composition was blended according to the following formulation, and tabletted in conventional manner.

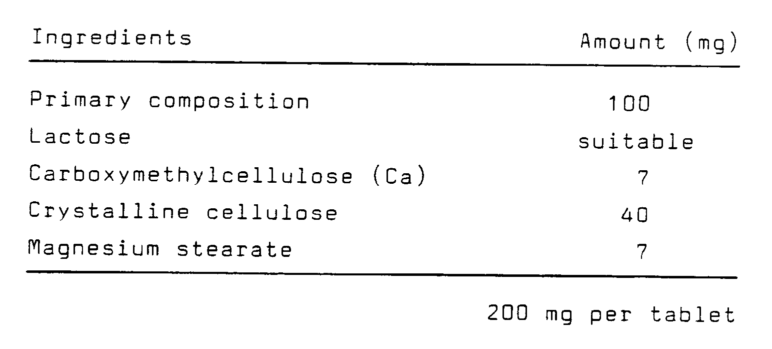

### Preparation Example 3

The primary composition was blended with a vehicle according to the following formulation, and encapsulated in conventional manner.

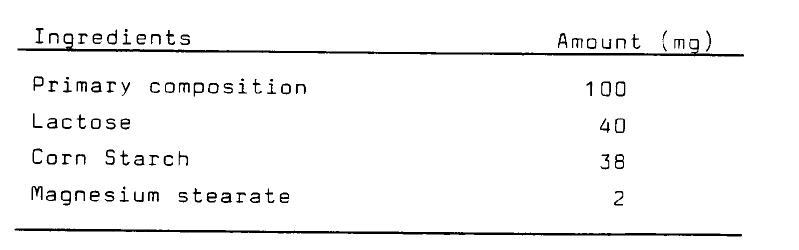

### Toxicity Tests

Acute and subacute (chronic) toxicity tests were performed using SD rats and mice which had received 3-OGP (referred to as Compound a) and the above-mentioned primary composition ( Composition b ). The results are reported below.

For experimentation, a group of 8-10 animals were used.

### (1) Acute Test

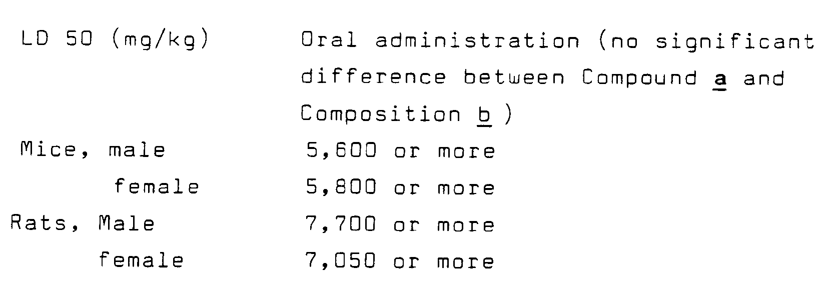

Between Compound a and Composition b there was no significant difference in acute toxicity, but the group which received Compound a alone showed general symptoms of appeasement, diarrheoa, vomiting and typhlectasis. The group which received Composition b, on the other hand, did not substantially have any particular symptom.

### (2) Subacute Test

Over 3 months, Compound a and Composition b were orally administered to two groups of SD male rats at doses of 256, 280, 640, 1300, 1600 and 4000 mg/kg/day to determine which dose had no toxic influence at all and which dose induced positive poisoning.

Compound a had no toxic influence whatsoever at 256 mg/kg but induced positive poisoning at 1600 mg/kg. Some animals were sacrificed at 400 mg/kg. Toxic symptoms then disappeared after no drug treatments over 5 weeks.

Composition b had no toxic influence whatsoever at 380 mg/kg but induced positive poisoning at 1300 mg/kg. No animals were sacrificed.

### Pharmaceutical Tests

Continuous 5-day administration of streptozocin (STZ) to an AKR/J1 line of mice, that is inbred mouse with a spontaneous symptom of leukemia, can cause it to show a disease similar to human Type I diabetes (dependent upon insulin). Tests were performed for the purpose of showing the effects Compound a and Composition b have on such mice with streptozosin-induced diabetes.

In the accompanying drawings:
Figures 1(a) and 1(b) are graphs showing the concentration of glucose in the blood and the amount of insulin in the pancreas measured with a mouse in accordance with Example 1,
Figures 2(a) and 2(b) are graphs showing the concentration of glucose in the blood and the amount of insulin in the pancreas measured with a mouse in accordance with Example 2, and
Figures 3(a) and 3(b) are graphs showing the concentration of glucose in the blood and the amount of insulin in the pancreas measured with a mouse in accordance with Example 3.

### Example 1

### (a) Procedures

Streptozocin (STZ) was continuously adminstered to the abdominal cavity of a (masculine) mouse belonging to AKR/J1 at a dose of 40 mg/kg for 5 days to induce an increase in the concentration of glucose in the blood and a decrease in the amount of insulin in the pancreas.

With Compound a dissolved in a phosphate buffer solution with pH 7.4, the concentration of glucose in the blood was measured at 7 days, 14 days and 21 days from STZ dosing, using a GOD-PAP kit (made by Boehringer Mannheim) based on the glucose oxidase process for measurement. In addition, the amount of insulin in the pancreas, extracted by the acid-ethanol process, was assayed with a radio active immuno assay kit (made by Pharmacia Aktiebolag) based on an immunoassay process making use of a competitive reaction by ¹²⁵I-labeled insulin (see Soeldner, JS & Slone, D, "Diabetes", Vol. 14, pp. 771-779, 1985).

### (b) Results

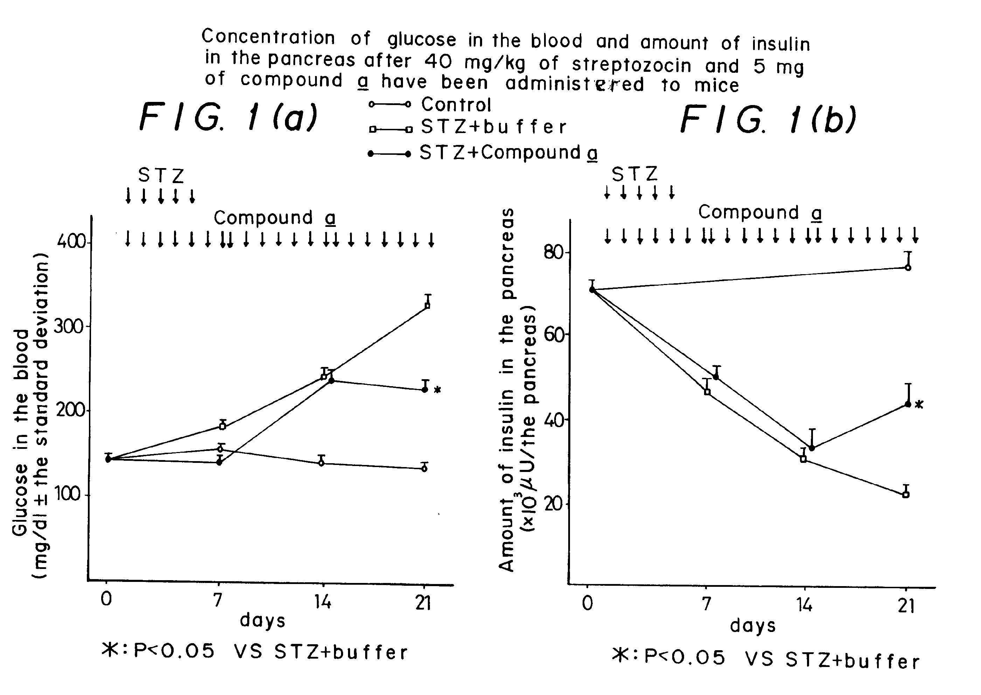

The concentration of glucose in the blood and the amount of insulin in the pancreas, as measured, are shown in the graphs depicted in Figures 1(a) and 1(b). The graphs indicate that at 21 days from dosing, Compound a could give rise to significant improvements in the concentration of glucose in the blood increased and the amount of insulin in the pancreas decreased by the administration of STZ at a rejection rate of 5%.

### Example 2

### (a) Procedures

A similar mouse was caused to develop STZ-induced diabetes in a similar manner as referred to in Example 1.

A solution of Compound a in a phosphate buffer with pH 7.4 was continuously adminstered to the abdominal cavity of the animal at 5 mg/kg over 7 days from STZ dosing.

At 7 days, 14 days and 21 days from STZ dosing, the concentration of glucose in the blood and the amount of insulin in the pancreas were measured in similar manners as mentioned in Example 1.

### (b) Results

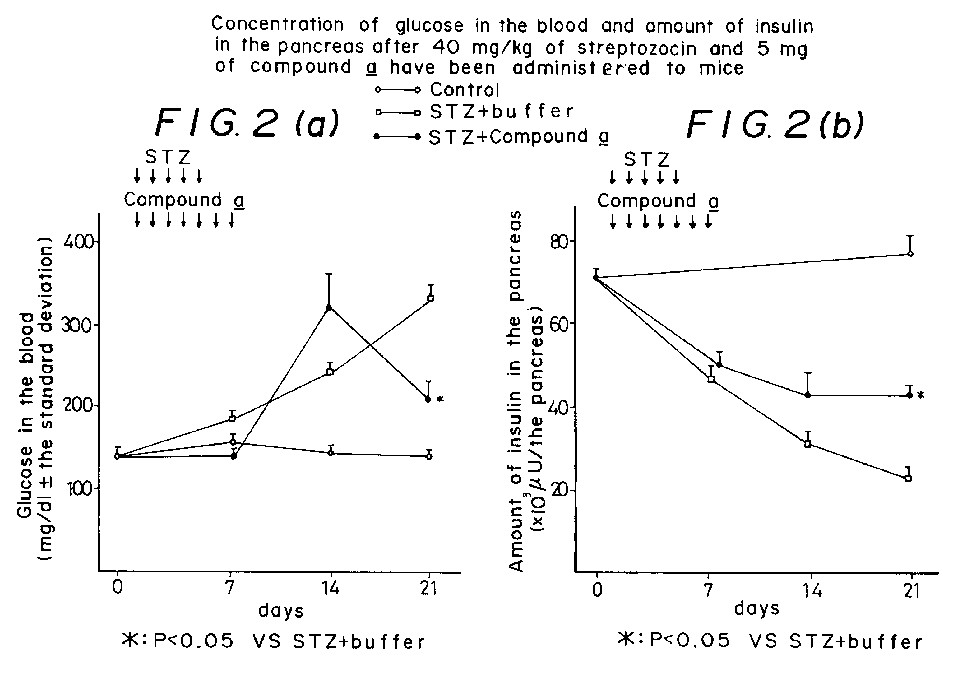

The concentration of glucose in the blood and the amount of insulin in the pancreas, as measured, are shown in the graphs depicted in Figures 2(a) and 2(b). The graphs indicate that at 21 days from dosing, Compound a could give rise to significant improvements in the concentration of glucose in the blood increased and the amount of insulin in the pancreas decreased by the administration of STZ at a rejection rate of 5 %.

### Example 3

### (a) Procedures

A similar mouse was caused to develop STZ-induced diabetes in a similar manner as referred to in Example 1.

A solution of Compound a in a phosphate buffer with pH 7.4 was continuously adminstered to the abdominal cavity of the animal at 5 mg/kg over 15 days from the 7th day after STZ dosing.

At 7 days, 14 days and 21 days from STZ dosing, the concentration of glucose in the blood and the amount of insulin in the pancreas were measured in similar manners as mentioned in Example 1.

### (b) Results

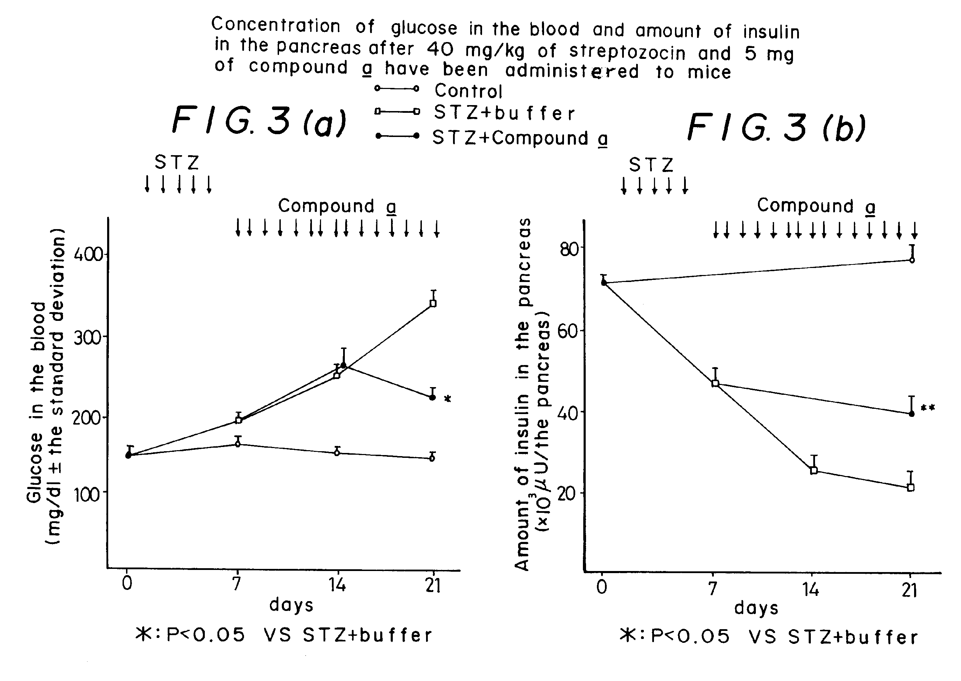

The concentration of glucose in the blood and the amount of insulin in the pancreas, as measured, are shown in the graphs depicted in Figures 3(a) and 2(b). The graphs indicate that at 21 days from dosing, Compound a could give rise to significant improvements in the concentration of glucose in the blood increased and the amount of insulin in the pancreas decreased by the administration of STZ at a rejection rate of 5 %.

### Example 4

### (a) procedures

Similar mice were caused to develop STZ-induced diabetes in a similar manner as described in Example 1.

A solution of Compound a in a similar phosphate buffer with pH 7.4 as used in Example 1 was continuously administered at a rejection rate of 5 % to the abdominal cavities according to similar dosing schemes as applied in Examples 1, 2 and 3.

At 21 days from STZ dosing, the degrees of cellular infiltration in the Langerhans' islands were assayed under an optical microscope.

### (b) Results

The degrees of cellular infiltration in the Langerhans′ islands, as assayed, are reported in Table 1. The results indicate that at 21 days from its dosing, Compound a successfully decreased the infiltrated cells increased by STZ dosing, ameliorating the conditions.

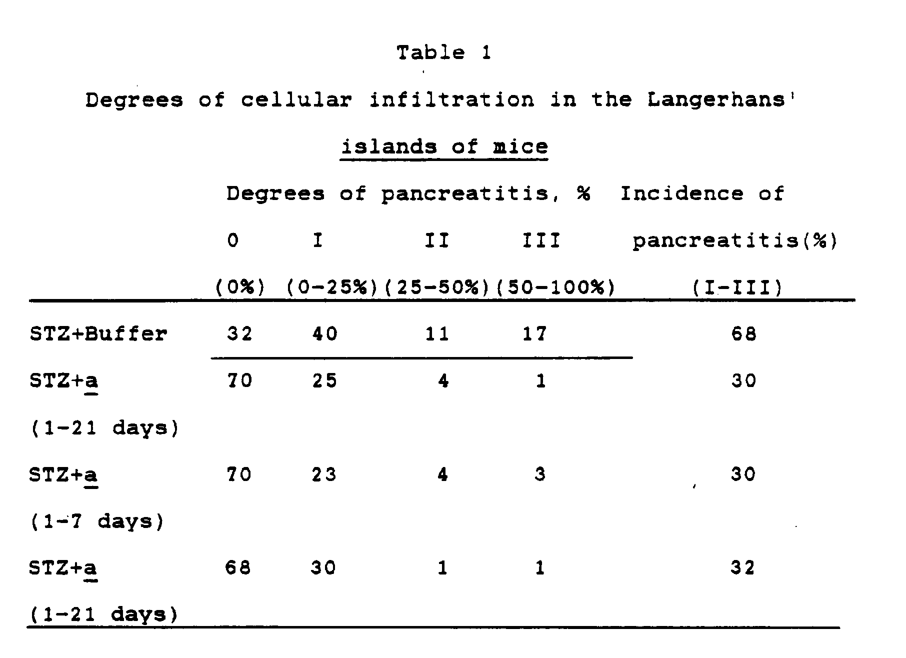

From the results of the pharmaceutical tests, it can be understood that the compositions used in this invention are greatly efficacious against STZ-induced diabetes similar to the human diabetes model. Thus, they can be used as an active ingredient for diabetes-treating drugs in humans.

## Claims

1. Use of 3-oxygermylpropionic acid for the manufacture of a medicament to treat or prevent diabetes mellitus due to autoimmune reactions, wherein said acid is used in a composition with a stabilizing carrier.

2. Use according to Claim 1, wherein said stabilizing carrier is hydroxypropylcellulose.

3. Use according to Claim 2, wherein the hydroxypropylcellulose is used in an amount of 2 parts per 1 part by weight of the 3-oxygermylpropionic acid.

4. Use according to any preceding claim, wherein the medicament is in a unit dosage form adapted to allow the administration of said acid in an amount of 5 mg/kg to 1500 mg/kg daily dose per human patient.

## Patentansprüche

1. Verwendung von 3-Oxygermylpropionsäure für die Herstellung eines Medikaments zur Behandlung oder Verhütung der Zuckerkrankheit durch Autoimmunreaktionen, wobei die benannte Säure in Verbindung mit einem stabilisierenden Träger eingesetzt wird.

2. Verwendung gemäss Anspruch 1, worin der benannte stabilisierende Träger Hydroxypropylcellulose ist.

3. Verwendung gemäss Anspruch 2, worin die Hydroxypropylcellulose in einem Gewichtsverhältnis von zwei Teilen pro einem Teil 3-Oxygermylpropionsäure eingesetzt wird.

4. Verwendung gemäss jedem beliebigen der vorstehenden Ansprüche, worin das Medikament in Einheitsdosierungen vorliegt, die es gestatten, die benannte Säure in Tagesdosen von 5 bis 1500 mg/kg an kranke Menschen zu verabreichen.

## Revendications

1. Utilisation de l'acide 3-oxygermylpropionique pour la fabrication d'un médicament pour traiter ou prévenir le diabète sucré dû à des réactions auto-immunes, où ledit acide est utilisé dans une composition avec un vecteur stabilisant.

2. Utilisation selon la revendication 1, où ledit vecteur stabilisant est l'hydroxypropylcellulose.

3. Utilisation selon la revendication 2, où l'hydroxypropylcellulose est utilisée à raison de 2 parties pour 1 partie en poids d'acide 3-oxygermylpropionique.

4. Utilisation selon l'une quelconque des revendications précédentes, où le médicament est sous une forme galénique permettant l'administration journalière au patient humain de quantités dudit acide allant de 5 mg/kg à 1500 mg/kg.
